# EUROPEAN PATENT APPLICATION

(11) **EP 1 402 824 A1**
(43) Date of publication of application: **31.03.2004**
(21) Application number: 02021455.7
(22) Date of filing: 25.09.2002
(51) Int. Cl.: A61B 17/04

(54) **Surgical stitching device for hepatic ligation**

(71) Applicant: Chang, Yu-Chung, Tainan City (TW)
(72) Inventor: Chang, Yu-Chung, Tainan City (TW)
(74) Representative: Söllner, Udo, Dipl.-Ing.

(57) **Abstract**

A hemostasis straight needle includes a sheath (10), an inner needle (20) reciprocally received in the sheath (10) and a plunger (50) partially received in the sheath (10) and connected to one end of the inner needle (20) for driving the inner needle (20) to penetrate a pathological organ for resection.

## Description

### BACKGROUND OF THE INVENTION

### 1. Field of the Invention

The present invention relates to a hemostasis straight needle, and more particularly to a hemostasis straight needle for hepatic resection.

### 2. Description of Related Art

Liver is an organ filled with blood. There are three major issues that the operation of hepatic resection has faced, which are: temporarily blocking the blood vessels without causing significant anoxaemia, minimizing the blood loss and determining the size of the portion of the liver to be removed. The conventional methods commonly used for hepatic resections are, now, as follow:
1. using Lin's clamp invented by Prof. T. Y Lin for achieving hemostasis by instrumental compression of the liver;
2. fragmenting the liver parenchyma by a vibration from ultrasonic fluid with only reserving blood vessels and ligating the blood vessels thereafter;
3. transecting the liver after clotting the blood in the blood vessels and tissue surrounding the top of a needle by a microwave coagulator; and
4. completing a resection after gelling the protein by the ultrahigh speed vibration with a harmonic scalpel and meanwhile achieving hemostasis.

The conventional methods popularly used for hepatic resections allow surgeons to complete the hepatic resections smoothly. However, each of the methods has its disadvantages and inconvenience respectively, wherein the shortcomings are, for example, increasing the training time needed for surgeons due to complexity of surgical techniques, and utilizing the complicated and expensive instruments, etc.

### SUMMARY OF THE INVENTION

The main objective of the present invention is to provide a simple hemostasis straight needle for hepatic resection that can make the hepatic resections easier and safer thereby contributing to the well-beings of the patients.

To achieve the objective, the hemostasis straight needle in accordance with the present invention comprises a sheath, an inner needle reciprocally received in the sheath and a plunger partially received in the sheath and connected to one end of the inner needle for driving the inner needle.

Further benefits and advantages of the present invention will become apparent after a careful reading of the detailed description with appropriate reference to the accompanying drawings.

### BRIEF DESCRIPTION OF THE DRAWINGS

Fig. 1 is a side cross-sectional plan view of a hemostasis straight needle for hepatic resection in accordance with the present invention;
Fig. 1A is a diagram illustrating the details of the hemostasis straight needle for hepatic resection and the local enlargement of the portion near the top of the inner needle;
Fig. 1B is a view of the plunger of the stainless steel inner needle inside the hemostasis straight needle in Fig. 1, wherein the plunger has a notch;
Fig. 1C is another view of the plunger of the stainless steel inner needle inside the hemostasis straight needle in Fig. 1, wherein the plunger has a groove;
Fig. 1D is another view of the plunger of the stainless steel inner needle inside the hemostasis straight needle in Fig. 1, wherein the plunger has a boss;
Fig. 2A is a schematic view showing the operation method of the hemostasis straight needle in Fig. 1, wherein the hemostasis straight needle has penetrated into the bottom of the liver and caught one end of a suturing thread;
Fig. 2B is another schematic view showing the operation method of the hemostasis straight needle in Fig. 1, wherein the suturing thread has been bringing up to the surface of the liver by pulling out the hemostasis straight needle from the bottom of the liver;
Fig. 2C is another schematic view showing the operation method of the hemostasis straight needle in Fig. 1, wherein the liver shown is a cross-sectional view and the hemostasis straight needle has penetrated into the bottom of the liver and caught one end of the suturing thread;
Fig. 2D is another schematic view showing the operation method of the hemostasis straight needle in Fig.1 from another direction, wherein the liver shown is a cross-sectional view, and hemostasis straight needle has brought one end of the suturing thread to the surface of the liver;
Fig. 2E is another schematic cross-sectional view showing the operation method of the hemostasis straight needle in Fig. 1 from another direction, wherein the hemostasis straight needle penetrates again into the bottom of the liver and catches the other end of the suturing thread, and two opposite ends of the suturing thread are tied with a knot after this procedure; and
Fig. 2F is a diagram showing two rows of interlocked sutures by using the hemostasis straight needle in accordance with the present invention.

### DETAILED DESCRIPTION OF THE INVENTION

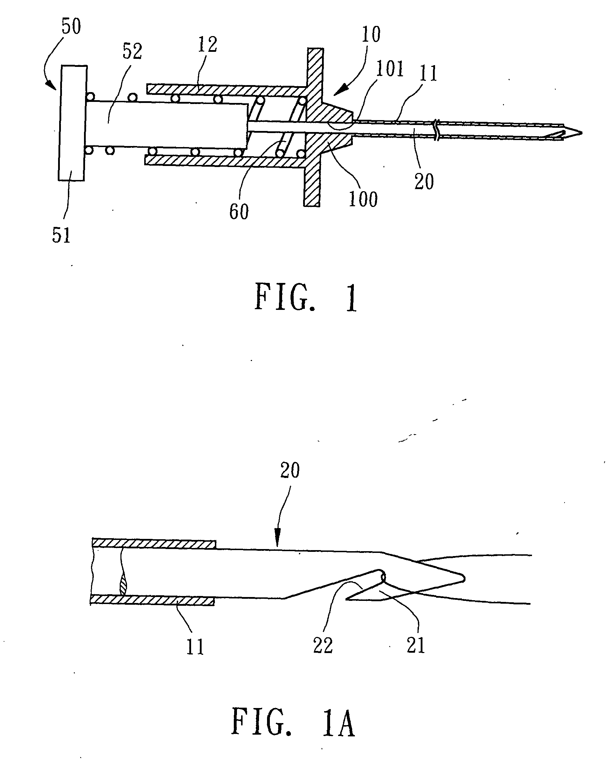

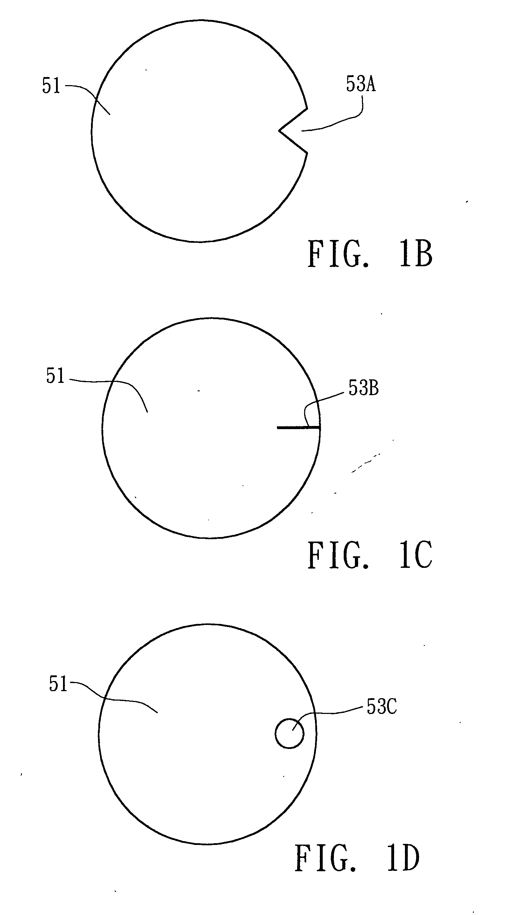

Referring to the drawings and initially to Figs. 1 and 1A, a hemostasis straight needle for hepatic resection in accordance with the present invention comprises a sheath (10), an inner needle (20) reciprocally received in the sheath (10) and a plunger (50) partially received in the sheath (10) and connected to one end of the inner needle (20).

The sheath (10) includes a body (100) and a through hole (101) longitudinally defined in the body (100). A tubular structure (11) extends from a first side of the body (100) along an axis of the through hole (101) in the body (100) and a cylinder (12) is formed on a second side of the body (100) along the axis of the through hole (101) in the body (100). The through hole (101) in the body (100) centrally communicates with an inner periphery of the tubular structure (11) and the cylinder (12). In the preferred embodiment of the present invention, the sheath (10) is made of stainless steel or plastic material.

The inner needle (20) is movably received in the tubular structure (11) and the cylinder (20). The inner needle (20) includes a first end selectively extending out of the tubular structure (11) and a second end received in the cylinder (12). The first end of the inner needle (20) is sharpened and has a hook (21) formed near a distal point of the first end of the inner needle (20) for defining a groove (22) in the first end of the inner needle (20). In the preferred embodiment of the present invention, the inner needle (20) is made of stainless steel.

The plunger (50) includes a button (51) and a stub (52) extending from the button (51) and reciprocally received in the cylinder (12). The stub (52) is securely connected to the second end of the inner needle (20) to reciprocally drive the inner needle (20) and make the hook (21) and the groove (22) selectively extending out of the tubular structure (11). A spring (60) is compressibly received in the cylinder (12) and mounted around the stub (52) of the plunger (50). The spring (60) has a first end abutting the second side of the body (100) and a second end abutting the button (51) of the plunger (50) such that the sharpened first end of the inner needle (20) extending out of the tubular structure (11) and the groove (22) is remained in the tubular structure (11) in an original condition.

With reference to Figs. 1B, 1 C and 1D, for indicating the direction in which the hook (21) hidden in the tubular structure (11) faces or under the liver, an indicating member is formed near a periphery of the button (51) opposite to the stub (52). The indicating member can be designed in various types, such as a notch (53A) shown in Fig. 1B, a groove (53B) shown in Fig. 1 C or a boss (53C) shown in Fig. 1 D.

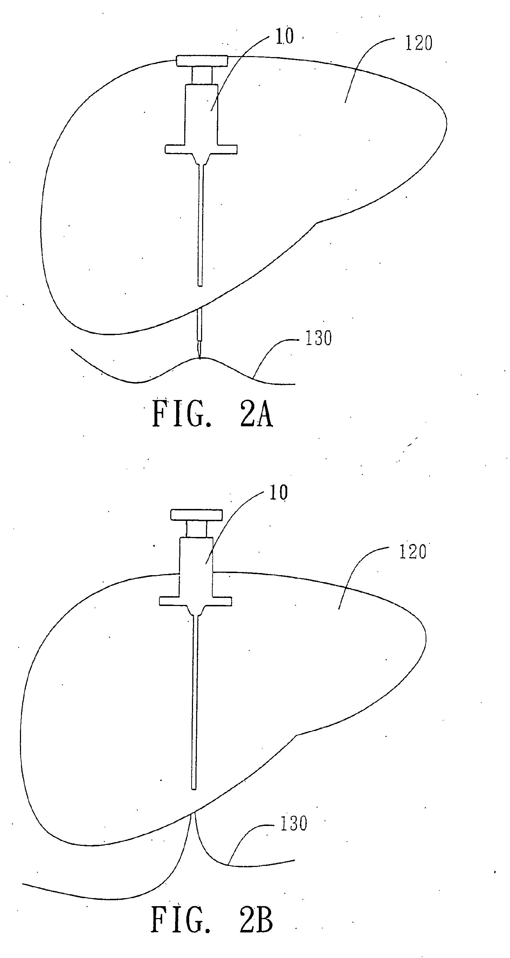

The operation method of the hemostasis straight needle for hepatic resection in accordance with the present invention is, now, as follows. After the abdomen is opened with a proper method, the ligaments surrounding the portion of the liver to be transacted have to be cut off. Then, by pushing down the hemostasis straight needle of the present invention while holding the inner needle (20) and the sheath (10) together, the tubular structure (11) with the inner needle (20) penetrates vertically into the bottom of the liver from the surface of the liver, and catches one end of a suturing thread with the hook (21) while the plunger (50) is further pushed down, wherein the suturing thread can be No. 1 silk but not limited thereto. Fig. 2A is a schematic view showing the operation method of the hemostasis straight needle according to a preferred embodiment of the present invention, wherein the hemostasis straight needle has penetrated into the bottom of the liver (120) and caught a suturing thread (130) with the hook (21). Then, the hook (21) is withdrawn into the sheath (10) by releasing the plunger (50) so as to pull out the hemostasis straight needle of the present invention without harming or damaging the vessels inside the liver (120). Fig. 2B is another schematic view showing the operation method of the hemostasis straight needle according to the preferred embodiment of the present invention, wherein the suturing thread (130) has been bringing out to the surface of the liver (120) by pulling out the hemostasis straight needle of the present invention.

Thereafter, the aforementioned steps for penetrating the hemostasis straight needle of the present invention into the liver (120) are repeated, and the locations penetrated currently and previously are on the same side of the predetermined division line, but are apart from 2 cm to 3 cm. After the hemostasis straight needle of the present invention penetrates, the other end of the suturing thread (130) is caught by the hook (21) and pulled out to the surface of the liver (120). Then, two ends of the suturing thread (130) on the liver surface are tied with a knot and thereby the blood vessels within the range of suturing thread (130) are totally ligated. Fig. 2C is another schematic view showing the operation method of hemostasis straight needle from the other direction according o the preferred embodiment of the present invention, wherein the liver (120) is illustrated with a cross-section view, and the hemostasis straight needle of the present invention has penetrated into the bottom of the liver (120) and caught one end of the suturing thread (130) with the hook (21). Fig. 2D is also another schematic view showing the operation method of the hemostasis straight needle of the present invention from the other direction, wherein the liver (120) is illustrated with a cross-section view, and the hemostasis straight needle of the present invention has brought up one end of the suturing thread (130) to the surface of the liver (120). Fig. 2E is also another schematic view showing the operation method of the hemostasis straight needle of the present invention from the other direction, wherein the liver (120) is illustrated with a cross-section view, and the hemostasis straight needle of the present invention has penetrated again into the bottom of the liver (120) and caught the other end of the suturing thread (130) with the hook (21).

After the aforementioned steps are repeated for a plurality of times along two sides of the predetermined divisional line, the interlocking sutures are formed along two sides of the predetermined division line for the whole liver parenchyma. With reference to Fig. 2F, Fig. 2F is a diagram showing two rows of finished interlocking sutures by using the hemostasis straight needle according to the preferred embodiment of the present invention, wherein two rows of interlocking sutures have been completed with a plurality of suturing threads (130) along two sides of the predetermined division line (140) of the liver (120). Furthermore, in order to firmly ligate all the related blood vessels as many as possible, two adjacent suturing threads (130) located on the same side of the predetermined division line (140) have to be properly interlocked. The length of the interlock (150) is about, for example, 0.5 cm to 1.0 cm.

Thereafter, along the predetermined division line in the middle of two rows of suturing threads (130), the liver (120) parenchyma is divided by electrocautery with minimal hemorrhage. For the safe's sake, during the operation of resection, the bigger blood vessels encountered are suture-ligated for reinforcement.

In September 1997, the hemostasis straight needle of the present invention was first successfully applied on a cirrhotic patient with hepatocellular carcinoma (HCC), right hepatic lobectomy was performed with minimal blood loss during the operation. From then on, there were forty-three cases as shown in Table 1, wherein nineteen HCCs, four cholangiocarcinomas (CCC), four colon metastases, one angiomyolipoma, one hemangioma, one liver trauma and thirteen intrahepatic duct (IHD) stones, and the hepatic resections performed include nine right lobectomies, five bisegmentectomies, seven segmentectomies, four subsegmentectomis, two partial hepatectomies, fifteen left lateral segmentectomies and one hepatorrhaphy. Ten patients had mild to severe degrees of liver cirrhosis (LC), one patient had chronic active hepatitis, two patients had liver fibrosis, and the other thirty patients each had a normal liver. However, two of the four patients with CCC showed marked cholestasis, and one of theses showed signs of sepsis before the operation.

Table 1 lists the forty-three cases using the hemostasis straight needle of the present invention. The scope of the present invention used in hepatic resections comprises: right lobectomy, right superior bisegmentectomy, right posterior bisegmentectomy, right posterosuperior segmentectomy, right posteroinferior segmentectomy, right inferior bisegmentectomy, right partial hepatectomy, and left lateral segmentectomy or partial hepactectomy. Except for the aforementioned hepatectomies, the present invention can also be used for segmental and partial hepactectomy of the left lateral segment, and partial hepatectomies along the inferior border of the liver under a window with gasless-fashioned laparoscopic surgery.

**Table 1.**

| is Patient's backgrounds and results of using the present invention. | | | | | | |
|---|---|---|---|---|---|---|
| Cases | Primary Diseases | Associated Liver Diseases | Operation | Blood Loss (ml) | Procedure Related Morbidity | Remark |
| 2 | HCC | Mild LC | Rt. Lobectomy | Minimal | No | |
| 1 | HCC | Mild LC | Rt. Lobectomy | 2200 | No | Unsecured Tie |
| 4 | HCC(14,10,20, 12cm) | No | Rt. Lobectomy | 1100,1500,650, 250 | No | Upper/Lower 1/3 not Applied |
| 1 | CCC | No | Rt. Lobectomy | 500 | Bile Leak | |
| 1 | IHD Stone | No | Rt. Lobectomy | Minimal | No | Atrophied Liver |
| 1 | HCC | Mod. LC | Bisegmentectomy | Minimal | No | S7, 8 |
| 2 | HCC | No | Bisegmentectomy | Minimal | No | S5, 6 |
| 1 | Colon Meta. | No | Bisegmentectomy | Minimal | No | S7,8 |
| 1 | AML | No | Bisegmentectomy | Minimal | No | S7,8 |
| 2 | HCC | Mod. LC | Segmentectomy | 100,150 | No | S6 |
| 1 | HCC | Fibrosis | Segmentectomy | 100 | No | S7 |
| 1 | HCC | CAH | Segmentectomy | Minimal | No | S6 |
| 2 | Colon Meta. | No | Segmentectomy | Minimal | No | S7 |
| 1 | Hemangioma | No | Segmentectomy | 100 | No | S7 |
| 2 | HCC | Fibrosis/No | Subsegmentectomy | Minimal | No | S6, S7 |
| 1 | HCC | Mild LC | Subsegmentectomy | 125 | No | S6-7 |
| 1 | CCC | Severe LC | Subsegmentectomy | Minimal | No | S7 |
| 2 | HCC | Mild/mod. LC | Partial Hepatectomy | Minimal | No | S6, S7 |
| 10 | IHD Stone | No | LLS | Minimal 1, | Bile leak | 2, wound Infections |
| 2 | IHD Stone | No | LLS | 75, 300 | No | |
| 1 | CCC | Liver Abscess | LLS | 1000 | No | Septic Coagulopathy |
| 1 | CCC | No | LLS | 75 | No | |
| 1 | Colon Meta. | No | LLS | 350 | No | |
| 1 | Trauma | No | Hepatorrhapy (rt.lobe) | Minimal | No | Died of Other Injuries |
| AML: Angiomyolipoma; CAH: Chronic Active Hepatitis | | | | | | |
| CCC: Cholangiocarcinoma; Colon Meta: Colon Metastasis; | | | | | | |
| HCC: Hepatocellular Carcinoma; IHD: Intra-Hepatic Duct; | | | | | | |
| LC: Liver Cirrhosis; LLS: Left Lateral Segmentectomy; | | | | | | |
| Mod: Moderate | | | | | | |

As described above, the hemostasis straight needle for hepatic resection in accordance with the present invention has several advantages as follow.
1. The hemostasis straight needle for hepatic resection of the present invention can make the hepatic resections safer thereby contributing to the well-beings of the patients worldwide.
2. The present invention is to simplify the surgical technique for hepatic resection, and further to reduce the training time needed for surgeons. Thus, the non-specialized general surgeons are able to perform hepatic resections easily, so that hepatic resections can be more popular all over the world.
3. The structure of the present invention is simple in mechanism, light and handy, small in size and low in price.
4. The present invention can be repeatedly sterilized and used.

Although the invention has been explained in relation to its preferred embodiment, it is to be understood that many other possible modifications and variations can be made without departing from the spirit and scope of the invention as hereinafter claimed.

## Claims

1. A hemostasis straight needle for hepatic resection, comprising:
a sheath;
an inner needle movably received in the sheath, the inner needle including a first end extending out of the sheath and being sharpened, the inner needle having a hook formed near a distal point of the first end for defining a groove in the first end of the inner needle;
a plunger connected to a second end of the inner needle to reciprocally drive the inner needle and make the hook and the groove selectively extending out of the sheath; and
a spring mounted around the plunger, the spring having a first end abutting the sheath and a second end abutting the plunger such that the first end of the inner needle extends out of the sheath and the groove is remained in the sheath in an original condition of the hemostasis straight needle.

2. The hemostasis straight needle as claimed in claim 1, wherein the sheath comprises a body and a through hole longitudinally defined in the body, a tubular structure extending from a first side of the body along an axis of the through hole and a cylinder formed on a second side of the body along the axis of the through hole in the body, wherein the through hole centrally communicated with an inner periphery of the tubular structure and the cylinder.

3. The hemostasis straight needle as claimed in claim 2, wherein the second end of the inner needle is movably received in the cylinder.

4. The hemostasis straight needle as claimed in claim 3, wherein the plunger comprises a stub partially and reciprocally received in the cylinder and securely connected to the second end of the inner needle.

5. The hemostasis straight needle as claimed in claim 4, wherein the plunger comprises a button and the stub extends from the button such that the first end of the spring abuts the second side of the body of the sheath and the second end of the spring abuts the button of the plunger.

6. The hemostasis straight needle as claimed in claim 5, wherein the plunger comprises an indicating member formed on the button opposite to the stub for indicating a direction when the hook is hidden in the tubular structure and under the liver.

7. The hemostasis straight needle as claimed in claim 6, wherein the indicating member is a groove defined in the periphery of the button.

8. The hemostasis straight needle as claimed in claim 6, wherein the indicating member is a notch defined in the periphery of the button.

9. The hemostasis straight needle as claimed in claim 6, wherein the indicating member is a boss extending from the button opposite to the stub.

## Amended claims

### Amended claims in accordance with Rule 86(2) EPC.

**1.** A hemostasis straight needle for hepatic resection, comprising:
- a sheath (10) incorporating a tubular structure (11);
- an inner needle (20) movably received in the sheath (10), the inner needle (20) including a first end extending out of the sheath (10) and being sharpened, the inner needle (20) having a hook (21) formed near a distal point of the first end for defining a groove (22) in the first end of the inner needle (20); and
- a spring (60) loaded device to reciprocally drive the inner needle (20) and make the hook (21) and groove (22) selectively extending out of the sheath (10);
**characterized in that**
the spring (60) loaded device is a plunger (50) connected to a second end of the inner needle (20), the spring (60) being mounted around the plunger (50) and having a first end abutting the sheath (10) and a second end abutting the plunger (50) such that the first end of the inner needle (20) extends out of the sheath (10) and the groove (22) is remained in the sheath (10) in an original condition of the hemostasis straight needle (20) and the plunger (50) having an indicating member (53A; 53B; 53C) indicating the direction in which the hook (21) is hidden in the tubular structure (11) of the sheath (10).

**2.** The hemostasis straight needle as claimed in claim 1, wherein the sheath comprises a body (100) and a through hole (101) longitudinally defined in the body (100), a tubular structure (11) extending from a first side of the body (100) along an axis of the through hole (101) and a cylinder (12) formed on a second side of the body (100) along the axis of the through hole (101) in the body (100), wherein the through hole (101) centrally communicates with an inner periphery of the tubular structure (11 ) and the cylinder (12).

**3.** The hemostasis straight needle as claimed in claim 2, wherein the second end of the inner needle (20) is movably received in the cylinder (102).

**4.** The hemostasis straight needle as claimed in claim 3, wherein the plunger comprises a stub partially and reciprocally received in the cylinder and securely connected to the second end of the inner needle.

**5.** The hemostasis straight needle as claimed in claim 4, wherein the plunger (50) comprises a button (51) and the stub (52) extends from the button (51) such that the first end of the spring (60) abuts the second side of the body (100) of the sheath (10) and the second end of the spring (60) abuts the button (51) of the plunger (50).

**6.** The hemostasis straight needle as claimed in claim 4, wherein the indicating member (53A; 53B; 53C) formed on the button (51) opposite to the stub (52) for indicating a direction when the hook (21) is hidden in the tubular structure (11) and under the liver.

**7.** The hemostasis straight needle as claimed in claim 5, wherein the indicating member is a groove (53B) defined in the periphery of the button (51).

**8.** The hemostasis straight needle as claimed in claim 5, wherein the indicating member is a notch (53A) defined in the periphery of the button (51).

**9.** The hemostasis straight needle as claimed in claim 5, wherein the indicating member is a boss (53C) extending from the button (51) opposite to the stub (52).
